(19) Europäisches Patentamt
European Patent Office
Office européen des brevets

(11) Numéro de publication: **0 102 908**
**A1**

(12) **DEMANDE DE BREVET EUROPEEN**

(21) Numéro de dépôt: **83420112.1**

(22) Date de dépôt: **04.07.83**

(51) Int. Cl.³: **C 07 D 513/04**
A 01 N 43/90
//(C07D513/04, 277/00, 239/00)

(30) Priorité: **23.07.82 FR 8213048**

(43) Date de publication de la demande:
**14.03.84 Bulletin 84/11**

(84) Etats contractants désignés:
**AT BE CH DE FR GB IT LI LU NL SE**

(71) Demandeur: **RHONE-POULENC AGROCHIMIE**
**14-20, rue Pierre Baizet**
**F-69009 Lyon(FR)**

(72) Inventeur: **Fabre, Jean-Louis**
**9, rue Fagon**
**F-75013 Paris(FR)**

(72) Inventeur: **Giraudon, Raymond**
**5, rue des Colverts**
**F-77330 Lesigny(FR)**

(72) Inventeur: **James, Claude**
**31 bis, Avenue Gambetta**
**F-75020 Paris(FR)**

(72) Inventeur: **Rubio, Claude**
**75, rue Duquesne**
**F-69006 Lyon(FR)**

(74) Mandataire: **Chaumette, Michel et al,**
**RHONE-POULENC AGROCHIMIE BP 9163-09**
**F-69263 Lyon Cedex 1(FR)**

(54) **Nouveaux dérivés de la tétrahydro-2,3,6,7 5H-thiaolo (3,2-a) pyrimidine, leur préparation et leur utilisation comme herbicides.**

(57) L'invention concerne de nouveaux dérivés de la tétrahydro-2,3,6,7 5H-thiazolo [3,2-a] pyrimidine. Ils répondent à la formule générale:

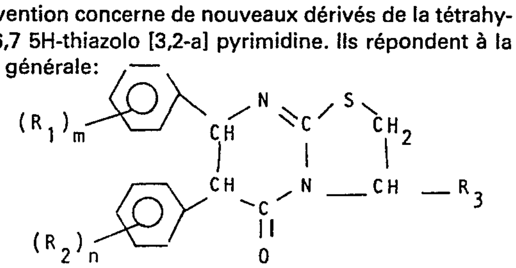

(I)

dans laquelle:

$R_1$ représente un halogène, un alkyle inférieur éventuellement halogéné, alkoxyle inférieur, alkylthio inférieur, nitro, cyano, alkylamino inférieur, amino dialkylamino, acétamido, R−CO, dans lequel R représente un alkyle inférieur éventuellement mono ou polyhalogéné, ou deux substituants $R_1$ forment ensemble un radical divalent alkylénedioxyle inférieur;

m est égal à un entier de 0 à 5;

$R_2$ représente un halogène ou un alkyle inférieur éventuellement mono ou polyhalogéné ou un alkoxyle inférieur, nitro ou cyano,

$R_3$ représente un hydrogène ou un alkyle inférieur,

n est égal à un entier de 0 à 3.

EP 0 102 908 A1

Croydon Printing Company Ltd

La présente invention concerne de nouveaux dérivés de l'oxo-5 tétrahydro-2,3,6,7 5H-thiazolo [3,2 - a] pyrimidine, leur préparation et leur utilisation comme herbicides dans le domaine agricole.

Plus précisément, l'invention a pour objet de nouveaux dérivés de l'oxo-5 tétrahydro-2,3,6,7 5H-thiazolo [3,2 - a] pyrimidine, utilisables comme herbicides sélectifs pour la destruction des adventices (ou mauvaises herbes) dans les grandes cultures, telles que le maïs, le riz, le tournesol et le coton.

L'un des buts de l'invention est précisément de mettre à la disposition de l'agriculture de nouveaux herbicides présentant une excellente activité herbicide vis-à-vis des mauvaises herbes, tout en étant parfaitement tolérés par les cultures concernées, à leur dose d'utilisation.

Certains dérivés de l'oxo-5 tétrahydro-2,3,6,7 5H-thiazolo [3,2 - a] pyrimidine ont déjà été décrits dans la littérature, toutefois, aucun ne l'a été en vue d'une utilisation comme herbicide en agriculture.

Ainsi, la demande de brevet européen n°81/401 178.9 publiée sous le n° 0 045 251 décrit, en tant que médicament utile pour le traitement de la maladie rhumatismale et comme anti-allergique le composé de formule

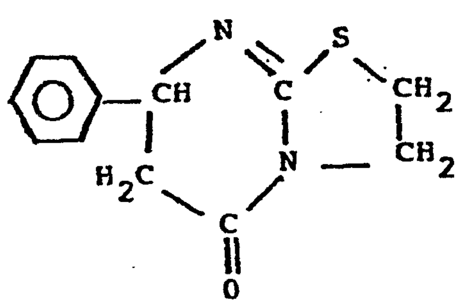

Les composés selon la présente invention sont différents de ceux décrits dans cette demande de brevet européen. Compte-tenu de cet art antérieur qui suggère des

BAD ORIGINAL

propriétés pharmaceutiques, les propriétés herbicides des composés selon la présente invention étaient sans aucun doute non prévisibles pour l'homme du métier.

Les composés selon la présente invention sont caractérisés en ce qu'ils répondent à la formule générale (I):

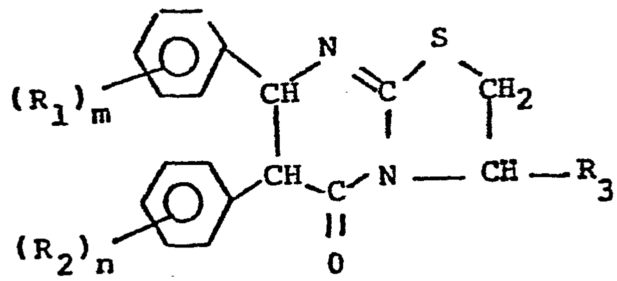

(I)

dans laquelle :

- $R_1$ représente un substituant choisi parmi les atomes d'halogènes (par exemple le fluor, le chlore, le brome) et les radicaux alkyles inférieurs, éventuellement mono ou polyhalogénés (par exemple le radical méthyle, éthyle, trifluorométhyle) ; alkoxyles inférieurs (par exemple méthoxyle) alkylthio inférieurs ; nitro ; cyano ; amino ; alkylamino inférieurs (par exemple méthylamino, éthylamino) ; dialkylamino dans lesquels chacune des parties alkyles, identiques ou différentes, comporte de 1 à 4 atomes de carbone ; acétamido et les radicaux acyles de formule R-CO- dans laquelle R représente un radical alkyle inférieur éventuellement mono ou polyhalogéné,

- ou, deux substituants $R_1$ forment ensemble un radical divalent alkylènedioxyle inférieur,

- m représente un nombre entier de 0 à 5, valeurs limites comprises, étant entendu que lorsque m est supérieur à 1, les substituants $R_1$ peuvent être soit identiques, soit différents,

BAD ORIGINAL

- $R_2$ représente un substituant choisi parmi les atomes d'halogènes (par exemple le fluor ou le chlore ou le brome) et les radicaux alkyles inférieurs éventuellement mono ou polyhalogénés ; alkoxyles inférieurs ; nitro et cyano,

- n représente un nombre entier de 0 à 3, valeurs limites comprises, étant entendu que, lorsque n est supérieur à 1, les substituants $R_2$ peuvent être soit identiques, soit différents.

- $R_3$ représente l'atome d'hydrogène ou un radical alkyle inférieur.

Au sens du présent texte, l'adjectif "inférieur", lorsqu'il qualifie un radical organique, signifie que ce radical comporte au plus six atomes de carbone, ce radical pouvant être soit linéaire, soit ramifié.

Les composés selon l'invention comportent tous deux carbones asymétriques respectivement en positions 6 et 7 sur l'hétérocycle, avec comme conséquence l'existence pour une formule chimique donnée de quatre formes stéréoisomères, regroupées en deux formes racémiques désignées dans ce qui suit par forme A et forme B et pouvant être différenciées par spectrographie RMN. Ces formes stéréoisomères ainsi que leurs mélanges sont compris dans le cadre de la présente invention.

Parmi les composés répondant à la formule (I), une sous-famille préférée du fait de ses bonnes propriétés herbicides est constituée par les composés pour lesquels $R_1$ représente un atome d'halogène ou un radical alkoxyle ou alkyle, comportant de 1 à 3 atomes de carbone, ou un radical alkylènedioxyle comportant de 1 à 2 atomes de carbone, m est égal à zéro, un ou deux, $R_2$ représente un radical alkyle comportant de 1 à 3 atomes de carbone et n est égal à zéro ou à 1, et $R_3$ représente l'atome d'hydrogène, ou le radical méthyle.

BAD ORIGINAL

Les composés selon l'invention, c'est-à-dire répondant à la formule (I), peuvent être préparés selon le procédé décrit ci-après qui est également compris dans le cadre de l'invention.

Ce procédé consiste à faire réagir le dérivé (chlorure ou ester) d'acide de formule (II), dans laquelle $R_1$, $R_2$, m et n ont la même signification que dans la formule (I), et $R_4$ représente un atome de chlore ou un radical alkoxyle inférieur (de préférence le radical méthoxyle ou éthoxyle),

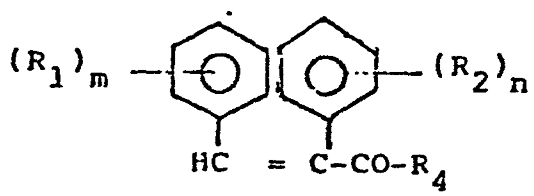

(II)

sur l'imino-2 thiazolidine de formule (III), ce composé étant en équilibre avec sa forme amino-2 thiazoline-2 de formule (IV) :

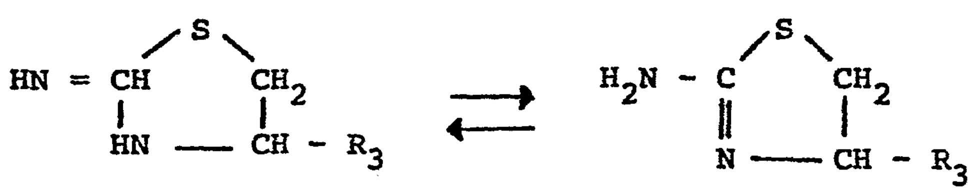

(III) (IV)

dans lesquelles $R_3$ a la même signification que dans la formule (I).

Selon que $R_4$ représente un atome de chlore ou un radical alkoxyle, les conditions à utiliser pour la réaction peuvent différer sensiblement.

Dans le cas où $R_4$ représente l'atome de chlore, la réaction s'effectue avantageusement à une température comprise entre moins 40°C et plus 160°C environ, dans un solvant inerte, c'est-à-dire ne réagissant pas vis-à-vis des réactifs

BAD ORIGINAL

en présence, de préférence anhydre, en présence d'un accepteur d'acide tel qu'une base minérale (par exemple un hydrogénocarbonate de métal alcalin) ou organique (par exemple la triéthylamine ou la pyridine ou l'imino-2 thiazolidine en excès utilisée alors à la fois comme réactif et accepteur d'acide).

Comme solvants utilisables pour la mise en oeuvre de cette réaction, on peut mentionner des hydrocarbures aliphatiques (par exemple n-heptane), ou aromatiques (par exemple toluène et xylènes) ; des hydrocarbures halogénés (par exemple chlorobenzènes, chloroforme, tétrachlorure de carbone, dichloro-1,2 éthane) ; des éthers (par exemple dioxanne, tétrahydrofuranne) ; des nitriles (par exemple l'acétonitrile) ; le diméthylformamide ; la N-méthyl pyrrolidone ; le diméthylsulfoxyde, etc...

Dans le cas où $R_4$ représente un radical alkoxyle inférieur, la réaction de l'ester de formule (II) avec l'imino-2 thiazolidine de formule (III), en équilibre avec sa forme (IV) s'effectue avantageusement à une température comprise entre plus 50°C et plus 160°C environ, en présence d'un agent antioxydant (par exemple l'hydroquinone), soit dans un solvant inerte, soit en l'absence de solvant. Comme solvants utilisables, on peut mentionner tout spécialement des hydrocarbures aromatiques tels que le benzène ou les xylènes ou le toluène, ou des hydrocarbures halogénés (chlorobenzène et dichlorobenzène) ou des hydrocarbures aromatiques nitrés (nitrobenzène par exemple). En fin d'opération, le composé de formule (I) peut être séparé du mélange réactionnel par tout moyen connu, par exemple par extraction au moyen d'un solvant et cristallisation, ou par chromatographie sur silice ou alumine.

Les composés de formules (II) et (III), utilisés comme matières de départ peuvent être préparés selon les méthodes décrites dans la demande de brevet européen n°

BAD ORIGINAL

81/401 178.9 citée plus haut. L'analyse par spectrographie RMN (résonance magnétique nucléaire) du composé de formule (I), tel qu'obtenu selon le procédé décrit plus haut, met en évidence, selon le cas, soit l'obtention d'une seule forme A ou B, telle que définie précédemment, soit l'obtention d'un mélange de ces deux formes A et B qui diffèrent entre elles par la valeur de la constante de couplage des deux protons portés par les carbones asymétriques situés en position 6 et 7 sur l'hétérocycle. Cette constante de couplage, exprimée en Hertz est représentée dans ce qui suit par $JH_{6-7}$. Par convention, dans le présent texte, on désignera par forme "A" celle des deux formes qui présente la constante de couplage $JH_{6-7}$ la plus élevée, cette constante étant en général supérieure à 7 Hz, et par forme "B" celle des deux formes qui présente la plus faible constante de couplage $JH_{6-7}$, cette constante étant en général inférieure ou égale à 7 Hz.

Ces deux formes "A" et "B" ainsi que leurs mélanges sont compris dans le cadre de l'invention. Il a été observé, d'après les essais déjà effectués, que, pour un composé de formule donnée, la forme "A" présente souvent une meilleure activité herbicide que la forme "B". Cette forme "A" est quelquefois obtenue directement par mise en oeuvre du procédé de préparation décrit plus haut. Toutefois, ce procédé conduit souvent soit à la forme "B" seule, soit à un mélange des formes A et B en proportions variables. La forme A peut être séparée à partir de ce mélange (chromatographie flash) ou par recristallisation dans un solvant approprié.

Dans le cas où le procédé de préparation décrit plus haut conduit à la forme B seule, il est possible de préparer la forme A, ou un mélange des formes A ét B, constitué essentiellement par la forme A, en traitant la forme B par une base, organique ou minérale, à une température comprise avantageusement entre plus 15°C et plus 80°C environ.

Comme base, on peut utiliser avantageusement soit un alcoolate de métal alcalin (par exemple l'éthylate de sodium), en opérant dans un alcanol (par exemple l'éthanol)

BAD ORIGINAL

ou dans un éther (par exemple le tétrahydrofuranne), soit la soude ou la potasse en opérant dans un alcanol.

Ce procédé qui permet la transformation de la forme B en la forme A, ou en un mélange des formes A et B d'un composé de formule donnée, ce mélange étant constitué essentiellement par la forme A, est également compris dans le cadre de l'invention. Il peut également être utilisé, en vue de transformer un mélange des formes A et B d'un composé de formule donnée, en un mélange de ces mêmes formes, constitué majoritairement par la forme A.

Les exemples décrits ci-après illustrent l'invention sans toutefois la limiter.

Parmi ces exemples, les exemples numérotés de 1 à 11 concernent la préparation des composés selon l'invention alors que ceux numérotés de I à III illustrent les propriétés herbicides de ces composés et leur sélectivité vis-à-vis des cultures. La structure des composés décrits ci-après a été caractérisée par spectrographie infra-rouge et par spectrographie RMN.

Exemple n° 1

Préparation de la forme B de la (chloro-4 phényl)-7 phényl-6 oxo-5 tétrahydro-2,3,6,7 5H-thiazolo [3,2-a] pyrimidine, (composé n° 1B), de formule :

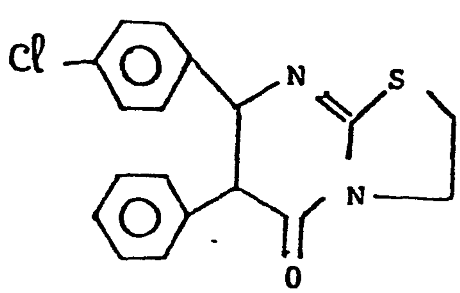

BAD ORIGINAL

A une solution de 10,4 g d'imino-2 thiazolidine dans 125 ml de chloroforme sont ajoutés 14,3 ml de triéthylamine puis on coule en 3 minutes une solution de 28,3g de chlorure de (chloro-4 phényl)-3 phényl-2 acryloyle dans 100 ml de chloroforme, à température ambiante (15 à 25°C).

Le mélange est chauffé à reflux pendant 18 heures environ puis concentré sous pression réduite. On traite avec 100 ml d'eau distillée et 50 ml d'acétate d'éthyle le résidu qui se délite et fournit après filtration et séchage 16,5 g de solide fondant à 188°C. Ce produit est purifié par chromatographie sur 300 g de silice (0,064-0,2 mm ; éluant : dichlorométhane 90 %, acétone 10 %) suivie d'une recristallisation dans 130 ml d'acétonitrile. On obtient 7,1g de cristaux blancs fondant à 201°C, analysés par spectrographie RMN comme la forme B ($JH_{6-7}$ = 6 Hz), désignée dans ce qui suit par composé n° 1B.

<u>Exemple n° 1bis</u>

<u>Préparation d'un mélange 55/45 des composés n° 1A et 1B</u>

A une solution de 8,8 g d'imino-2 thiazolidine dans 100 ml d'acétonitrile, on ajoute 12 ml de triéthylamine, puis on coule rapidement une solution de 23,9 g de chlorure de (chloro-4 phényl)-3 phényl-2 acryloyle dans 85 ml d'acétonitrile.

Le mélange réactionnel est chauffé à reflux pendant 18 heures, puis ramené à température ambiante (15 à 25°C) versé sur une solution aqueuse d'acide chlorhydrique 2N et agité pendant 10 minutes. La solution est ramenée à pH = 6 par addition d'une solution aqueuse de carbonate de sodium, puis extraite par 300 ml de dichlorométhane. La phase organique, séchée sur sulfate de sodium puis concentrée sous pression réduite, donne 26,9 g d'un produit brut que l'on purifie par recristallisation dans 20 ml de toluène suivie

BAD ORIGINAL

d'une chromatographie sur 220 g d'alumine (éluant : acétate d'éthyle). On obtient ainsi 7,1 g d'un solide blanc fondant à 155°C qui est analysé comme un mélange 55/45 des formes A et B (composé n° 1A et 1B) de la (chloro-4 phényl)-7 phényl-6 oxo-5 tétrahydro-2,3,6,7 5h-thiazolo [3,2-a] pyrimidine dont la formule est donnée dans l'exemple n° 1.

Composé n° 1A : ($JH_{6,7}$ = 10 Hz)

Composé n° 1B : ($JH_{6,7}$ = 6 Hz)

Exemple n° 1ter

Préparation du composé n° 1A à partir du composé n°1B

A une solution de 3,4 g du composé n° 1B, décrit dans l'exemple 1 dans 100 ml de tétrahydrofuranne, on ajoute 70 mg de tertiobutylate de potassium.

La solution agitée sous argon est portée à 30°C pendant 90 mn. On la maintient 18 heures sous agitation à température ambiante (15 à 25°C environ) puis on verse le mélange réactionnel sur 500 ml d'une solution aqueuse de chlorure d'ammonium servant à tamponner le milieu. On extrait avec 2 x 100 ml de dichlorométhane, sèche sur sulfate de sodium, filtre et concentre. Après purification par chromatographie sur silice (éluant : éther éthylique 99 %, éthanol 1 %), puis concentration des fractions, on obtient 2,8 g d'un produit que l'on cristallise dans l'éthanol.

Après filtration et sèchage, on obtient 2,7 g d'un produit fondant à 140°C, analysé par RMN comme un mélange 75/25 du composé n° 1A et du composé n° 1B.

Par recristallisation de 2 g de ce mélange dans 7ml d'éthanol, on recueille 1,2 g d'un produit fondant à 135°C, analysé par RMN comme étant la forme A de la (chloro-4 phényl)-7 phényl-6 oxo-5 tétrahydro-2,3,6,7, 5H-thiazolo [3,2-a] pyrimidine dont la formule est donnée dans l'exemple 1.

BAD ORIGINAL

Exemple n° 1 quarto

Préparation à partir du composé n° 1B, d'un mélange des composés n° 1A et 1B comprenant majoritairement le composé n° 1A.

A une solution de 5 g du composé n° 1B dont la préparation est décrite dans l'exemple n° 1, dans 250 ml d'éthanol, chauffée à 75°C, on ajoute 0,2 g de NaOH en solution dans 10 ml d'éthanol.

Le mélange réactionnel est agité pendant une heure en laissant la température revenir à l'ambiante, puis on verse le mélange réactionnel sur une solution de 5 g de chlorure d'ammonium dans 800 m d'eau. On extrait par 250 ml de dichlorométhane lave la phase organique avec 250 ml d'eau distillée, puis la sèche sur sulfate de sodium.

Par concentration sous pression réduite (40 mbar) ; on obtient 4,8 g d'un solide blanc que l'on fait cristalliser dans 20 ml d'éthanol.

Après filtration et séchage sous vide (1 mbar), on obtient 4,1 g d'un produit, fondant à 136°C analysé par RMN comme un mélange 85/15 du composé n° 1A ($JH_{6-7}$ = 10 Hz), et du composé n° 1B ($JH_{6-7}$ = 6 Hz) dont la formule est donnée dans l'exemple 1.

Exemple 2

Préparation des formes A et B (composés n° 2A et 2B) de la diphényl-6,7 oxo-5 tétrahydro-2,3,6,7 5H-thiazolo [3,2-a] pyrimidine, de formule :

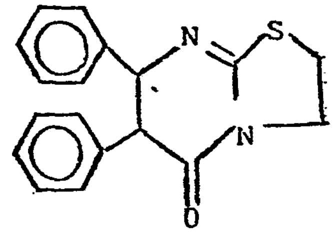

BAD ORIGINAL

A une solution de 9,9 g d'imino-2 thiazolidine dans 100 ml de chloroforme, on ajoute 11,6 ml de triéthylamine puis on coule en 5 minutes environ une solution de 20 g de chlorure de diphényl-2,3 acryloyle dans 80 ml de chloroforme.

Le mélange est chauffé à reflux pendant 18 heures, puis concentré sous pression réduite. On traite avec 100 ml d'eau distillée et 50 ml d'acétate d'éthyle le résidu pâteux qui se délite et fournit après filtration et séchage 19 g de produit solide fondant à 160°C. Ce produit est purifié par chromatographie sur 190 g de silice (éluant : acétate d'éthyle). Aux 12 g de solide blanc recueillis, on ajoute 3,8g d'un lot obtenu par une préparation similaire. Le mélange recristallisé dans 75 ml d'acétonitrile fournit 13 g de solide blanc fondant à 180°C et analysé par spectrographie RMN comme la forme A du composé dont la formule est donnée plus haut ($JH_{6-7}$ = 9 Hz), désignée dans ce qui suit par composé n° 2A.

En opérant selon des conditions opératoires légèrement différentes, on a obtenu un mélange des formes A et B du composé dont la formule est donnée au début de cet exemple, à partir duquel on a isolé par chromatographie, la forme B, fondant à 199°C, ($JH_{6-7}$ = 6 Hz), désignée par composé n° 2B.

<u>Exemple 3</u>

Préparation de la forme B de la (chloro-3 phényl)-7 phényl-6 oxo-5 tétrahydro-2,3,6,7 5H-thiazolo [3,2-a] pyrimidine (composé n° 3B), de formule :

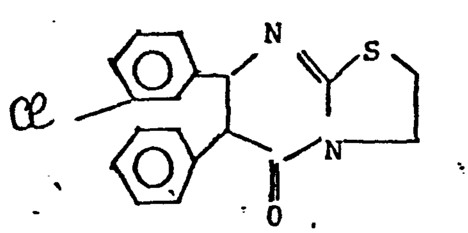

A une solution de 2,0 g d'imino-2 thiazolidine dans 60 ml d'acétonitrile, on ajoute 2,8 ml de triéthylamine. La solution est rapidement refroidie à moins 20°C et on coule

BAD ORIGINAL

alors, en une minute, une solution de 5,5 g de chlorure de (chloro-3 phényl)-3 phényl-2 acryloyle dans 20 ml d'acétonitrile, en maintenant la température inférieure à moins 10°C.

La suspension obtenue est agitée pendant une heure à une température comprise entre moins 5°C et moins 10°C, puis ramenée à l'ambiante, puis le chlorure de triéthylammonium qui a précipité est éliminé par filtration.

Après concentration du filtrat sous pression réduite, on obtient 6,5 g de solide blanc que l'on dissout dans 150 ml de dichlorométhane.

Le filtrat est lavé par deux fois 100 ml d'eau distillée et concentré sous pression réduite. On obtient 4,8g d'un produit blanc, fondant à 146-150°C, analysé par RMN comme étant la forme B ($JH_{6-7}$ = 7 Hz) du composé dont la formule est donnée dans cet exemple. Cette forme B est désignée par composé n° 3B.

Exemple n° 4

Préparation des formes A et B de la (chloro-2 phényl)-7 phényl-6 oxo-5 tétrahydro-2,3,6,7 5H-thiazolo [3,2-a] pyrimidine (composés n° 4A et 4B), de formule :

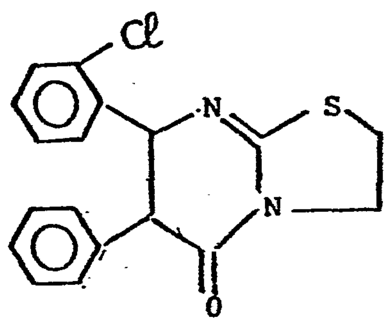

On dissout successivement dans 65 ml d'acétonitrile, chauffé à 60°C, 7,6 ml de triéthylamine, puis 5,5 g d'imino-2 thiazolidine. On coule ensuite en une minute une solution de 15 g de chlorure de (chloro-2 phényl)-3 phényl-2 acryloyle dans 56 ml d'acétonitrile, puis le mélange réactionnel est chauffé à reflux pendant 18 heures et concentre sous pression réduite.

BAD ORIGINAL

On dissout le résidu pâteux (24 g) dans 250ml de dichlorométhane, lave la solution obtenue avec 3 x 250 ml d'eau distillée, la sèche sur sulfate de sodium puis la concentre sous pression réduite.

Par recristallisation dans l'acétone, on obtient 12,3 g d'un solide blanc fondant à 179°C.

Par deux chromatographies successives, on sépare 4,9g d'un produit fondant à 180-181°C, analysé par RMN comme étant la forme B ($JH_{6-7}$ = 6 Hz), désigné dans ce qui suit par composé n° 4B, et 3,9 g d'un produit fondant à 177°C, analysé par RMN comme étant la forme A ($JH_{6-7}$ = 8 Hz) et désigné par composé n° 4A.

Exemple n° 5

Préparation des formes A et B (composés n° 5A et 5B) de la (méthoxy-4 phényl)-7 phényl-6 oxo-5 tétrahydro-2,3,6,7 5H-thiazolo [3,2-a] pyrimidine, de formule :

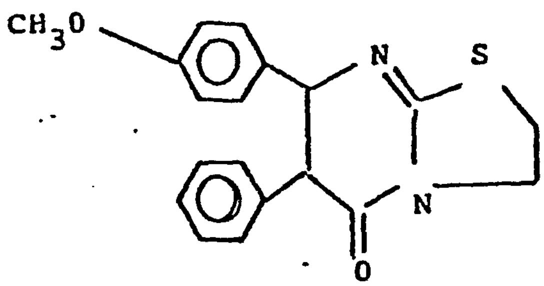

A une solution de 12,5 g d'imino-2 thiazolidine dans 135 ml de chloroforme, on ajoute 17 ml de triéthylamine puis on coule en une minute une solution de 33,3 g de chlorure de (méthoxy-4 phényl)-3 phényl-2 acryloyle dans 110ml de chloroforme. Le mélange est chauffé à reflux pendant 18 heures puis concentré sous pression réduite. On traite le résidu pâteux avec 100 ml d'eau et 50 ml d'acétate d'éthyle. Une huile épaisse décante que l'on dissout dans du dichlorométhane. La solution obtenue est lavée avec deux fractions de 200 ml d'une solution aqueuse d'acide chlorhydrique normale, puis avec trois fractions de 200 ml d'eau distillée avant d'être séchée sur sulfate de sodium.

BAD ORIGINAL

Après concentration et sèchage sous vide (2 mbar) à 40-50°C pendant environ 18 heures, on obtient 21,1 g d'un solide fondant à partir de 80°C.

Ce composé est analysé par RMN, comme un mélange 80/20 du composé n° 5A ($JH_{6-7}$ = 9 Hz) et du composé n° 5B ($JH_{6-7}$ = 5 Hz) dont la formule est donnée dans cet exemple.

Exemple n° 6

Préparation de la forme B de la (dichloro-3,4 phényl)-7 phényl-6 oxo-5 tétrahydro-2,3,6,7 5H-thiazolo [3,2-a] pyrimidine (composé n° 6B), de formule :

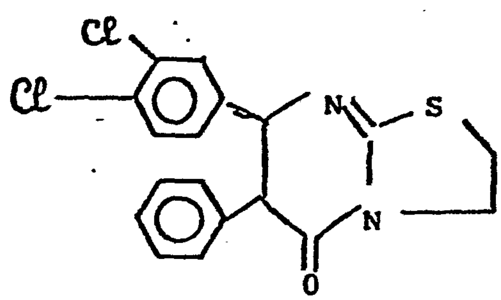

A une solution de 5,1 g d'imino-2 thiazolidine dans 60 ml de chloroforme, on ajoute 7,0 ml de triéthylamine puis on coule en une minute une solution de 15,6 g de (dichloro-3,4 phényl)-3 phényl-2 acryloyle dans 100 ml de chloroforme.

Le mélange est chauffé à reflux 20 heures, puis concentré sous pression réduite. On dissout le solide collant obtenu dans 200 ml de dichlorométhane. La solution est lavée avec deux fois 100 ml d'eau distillée, séchée sur sulfate de sodium et concentrée sous pression réduite. On obtient 13 g d'un produit beige que l'on recristallise dans 100 ml d'acétone.

On obtient alors un produit blanc, fondant à 167-171°C, analysé par RMN comme étant la forme B du composé dont la formule est donnée ci-dessus ($JH_{6-7}$ = 6 Hz), désigné par composé n° 6B.

BAD ORIGINAL

Exemple n° 7

Préparation de la forme A de la bis (chloro-4 phényl)-6, 7 oxo-5 tétrahydro-2,3,6,7 5H-thiazolo [3,2-a] pyrimidine (composé n° 7A), de formule :

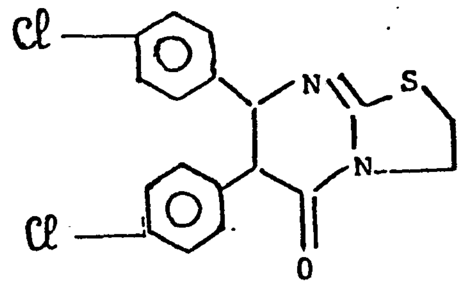

Dans 180 ml de diméthylformamide chauffé à 100°C, on ajoute 5,6 ml de triéthylamine puis, simultanément, 4,0 g d'imino-2 thiazolidine et 12,3 g de chlorure de bis (chloro-4 phényl)-2,3 acryloyle.

Le mélange réactionnel est maintenu 15 minutes à 100°C. On laisse la température revenir à l'ambiante puis on élimine, sous pression réduite (4 mbar) le diméthylformamide. Le produit brut obtenu (25,1g) est repris par 400 ml de dichlorométhane. On lave cette solution avec des fractions de 200 ml d'eau distillée puis d'une solution aqueuse de carbonate de potassium.

La phase organique séchée sur sulfate de sodium fournit après concentration sous pression réduite 9,1 g de solide beige que l'on traite par 40 ml d'acétonitrile chaud. Le solide blanc obtenu après cristallisation est recristallisé dans 15 ml d'acide acétique.

On obtient 3,2 g d'un produit blanc fondant à 232°C analysé par RMN comme étant la forme A du composé dont la formule est donnée plus haut, ($JH_{6-7}$ = 11 Hz), désigné par composé n° 7A.

BAD ORIGINAL

Exemple n° 8

Préparation de la forme B de la (méthyl-4 phényl)-7 phényl-6 oxo-5 tétrahydro-2,3,6,7 5H-thiazolo [3,2-a] pyrimidine (composé n° 8B), de formule :

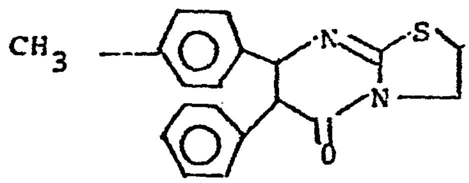

A une solution de 17 ml de triéthylamine dans 290ml d'acétonitrile, on ajoute 12,4 g d'imino-2 thiazolidine puis refroidit à moins 25°C. Sur la suspension ainsi obtenue, on coule 200 ml d'une solution de 31 g de chlorure de (méthyl-4 phényl)-3 phényl-2 acryloyle dans l'acétonitrile en 40 mn, le mélange réactionnel étant maintenu à une température inférieure ou égale à moins 20°C. On laisse ensuite revenir lentement la température à l'ambiante.

On chauffe ensuite 30 mn au reflux puis concentre sous pression réduite. Le solide pâteux obtenu (54,2 g) est dissous dans 600 ml de dichlorométhane, débarassé du chlorure de triéthylammonium par cinq extractions avec 150 ml d'eau chacune.

Après sèchage de la phase organique sur sulfate de sodium, filtration et concentration sous pression réduite, on obtient un produit que l'on fait cristalliser dans 250 ml d'éthanol à l'ébullition.

Après filtration et sèchage, on obtient 17,3 g d'un produit fondant à 193°C analysé par RMN comme la forme B du composé dont la formule est donnée plus haut ($JH_{6-7}$ = 6 Hz) et désigné par composé n° 8B.

BAD ORIGINAL

Exemple n° 8 bis

Préparation de la forme A de la (méthyl-4 phényl)-7 phényl-6 oxo-5 tétrahydro-2,3,6,7 5H-thiazolo [3,2-a] pyrimidine (composé n° 8A) à partir du composé 8B.

A une solution de 12,0 g du composé n° 8B (dont la préparation est décrite dans l'exemple n° 8), dans 300 ml d'éthanol, chauffée à 75°C, on ajoute 0,1 g de soude en solution dans 10 ml d'éthanol et agite deux heures en laissant la température revenir à l'ambiante. On verse ensuite le mélange réactionnel dans un litre d'eau, extrait par deux fois 200 ml de dichlorométhane et sèche les phases organiques rassemblées sur sulfate de sodium.

Par concentration sous pression réduite (40 mbar), on obtient 11,7 g de solide jaune pâle que l'on recristallise dans 100 ml d'éthanol. Après filtration et séchage sous vide (1 mbar), on obtient 8,2 g d'un produit blanc fondant à 118-120°C analysé par RMN, comme la forme A du composé dont la formule est donnée dans l'exemple 8 ($JH_{6-7}$=7Hz) et désigné par composé n° 8A.

Exemple n° 9

En opérant selon la méthode décrite dans l'exemple n° 8, à partir des matières premières appropriées, on prépare la forme B de la phényl-7 (méthoxy-4 phényl)-6 oxo-5 tétrahydro-2,3,6,7 5H-thiazolo [3,2-a] pyrimidine (composé n° 9B), de formule :

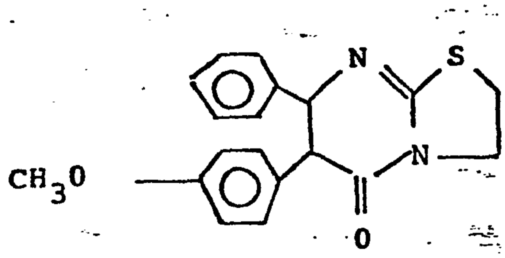

Point de fusion 171°C

($JH_{6-7}$ = 6 Hz)

BAD ORIGINAL

Exemple n° 10

Préparation de la forme B de la (nitro-4 phényl)-7 phényl-6 oxo-5 tétrahydro-2,3,6,7 5H-thiazolo [3,2-a] pyrimidine (composé n° 10B) de formule :

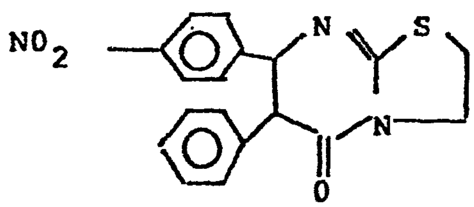

A une solution de 15,2 ml de triéthylamine dans 50ml de chloroforme, on ajoute 11,0 g d'imino-2 thiazolidine et on agite jusqu'à dissolution complète.

Dans cette solution refroidie à moins 30°C, on coule en 30 minutes une solution de 31,1 g de chlorure de (nitro-4 phényl)-3 phényl-2 acryloyle dans 100 ml de chloroforme, on maintient la température pendant une heure à moins 20°C, puis on laisse la température revenir à température ambiante (15 à 25°C) en une heure.

Le mélange réactionnel homogène est débarrassé du chlorure de triéthylammonium par trois extractions avec 200ml d'eau distillée.

Après séchage sur sulfate de sodium et concentration sous pression réduite, on obtient 35,4 g d'un produit orange que l'on recristallise dans 200 ml d'éthanol à l'ébullition.

Après recristallisation et séchage sous 1 mbar, on obtient 21,6 g d'un produit fondant à 194°C, analysé par RMN comme étant la forme B du composé dont la formule est donnée plus haut ($JH_{6-7}$ = 5 Hz) (composé n° 10B).

BAD ORIGINAL

Exemple n° 11

En opérant comme indiqué dans les exemples précédents, les composés ci-après ont été préparés : n° 6 A, 9 A, n° 11 A et 11 B : formes A et B de la (fluoro-2 phényl)-7 phényl-6 oxo-5 tétrahydro-2,3,6,7 5 H-thiazolo [3,2-a] pyrimidine,

n° 12 A : forme A de la (méthoxy-3-phényl)-7 phényl-6 oxo-5 tétrahydro-2,3,6,7 5 H-thiazolo [3,2-a] pyrimidine,

n° 13 A : forme A de la (bromo-3 phényl)-7 phényl-6 oxo-5 tétrahydro-2,3,6,7 5 H-thiazolo [3,2-a] pyrimidine,

n° 14 A + 14 B : mélange des formes A et B de la (méthyl-3 phényl)-7 phényl-6 oxo-5 tétrahydro-2,3,6,7 5 H-thiazolo [3,2-a] pyrimidine,

n° 15 A + 15 B : mélange de formes A et B de la (fluoro-3 phényl)-7 phényl-6 oxo-5 tétrahydro-2,3,6,7 5 H-thiazolo [3,2-a] pyrimidine,

n° 16 A : forme A de la (éthoxy-3 phényl)-7 phényl-6 oxo-5 tétrahydro-2,3,6,7 5 H-thiazolo [3,2-a] pyrimidine,

n° 17 A : forme A de la (fluoro-4 phényl)-7 phényl-6 oxo-5 tétrahydro-2,3,6,7 5 H-thiazolo [3,2-a] pyrimidine,

n° 18 A : forme A de la (bromo-4 phényl)-7 phényl-6 oxo-5 tétrahydro-2,3,6,7 5 H-thiazolo [3,2-a] pyrimidine,

n° 19 : forme A de la (cyano-4 phényl)-7 phényl-6 oxo-5 tétrahydro-2,3,6,7 5 H-thiazolo [3,2-a] pyrimidine,

n° 20 B : forme B de la (éthyl-4 phényl)-7 phényl-6 oxo-5 tétrahydro-2,3,6,7 5 H-thiazolo [3,2-a] pyrimidine,

n° 21 B : forme B de la (méthylthio-4 phényl)-7 phényl-6 oxo-5 tétrahydro-2,3,6,7 5 H-thiazolo [3,2-a] pyrimidine,

n° 22 A : forme A de la (méthylènedioxy-3,4 phényl)-7 phényl-6 oxo-5 tétrahydro-2,3,6,7 5 H-thiazolo [3,2-a] pyrimidine,

n° 23 A + 23 B : mélange des formes A et B de la (diméthyl-3,4 phényl)-7 phényl-6 oxo-5 tétrahydro-2,3,6,7 5 H-thiazolo [3,2-a] pyrimidine,

BAD ORIGINAL

n° 24 A + 24 B : mélange des formes A et B de la (diméthyl-2,4 phényl)-7 phényl-6 oxo-5 tétrahydro-2,3,6,7 5 H-thiazolo [3,2-a] pyrimidine,

n° 25 B : forme B de la phényl-7 (chloro-4 phényl)-6 oxo-5 tétrahydro-2,3,6,7 5 H-thiazolo [3,2-a] pyrimidine,

n° 26 A et 26 B : formes A et B de la (chloro-4 phényl)-7 (fluoro-4 phényl)-6 oxo-5 tétrahydro-2,3,6,7 5 H-thiazolo [3,2-a] pyrimidine,

n° 27 A et 27 B : formes A et B de la (méthyl-4 phényl)-7 (chloro-2 phényl)-6 oxo-5 tétrahydro-2,3,6,7 5 H-thiazolo [3,2a] pyrimidine,

n° 28 A et 28 B : formes A et B de la (méthyl-4 phényl)-7(chloro-3 phényl)-6 oxo-5 tétrahydro-2,3,6,7 5 H-thiazolo [3,2-a] pyrimidine,

n° 29 A et 29 B : formes A et B de la méthyl-3 (méthyl-4 phényl)-7 phényl-6 oxo-5 tétrahydro-2,3,6,7 5 H-thiazolo [3,2-a] pyrimidine.

Les formules de ces composés ainsi que leurs points de fusion sont indiqués dans le tableau (V) figurant à la fin de la description.

Les exemples ci-après, n° I, II, II bis et III et IV illustrent les propriétés herbicides des composés selon l'invention.

Exemple I - Activité herbicide en serre, en prélevée des espèces végétales

On prépare une suspension aqueuse ayant la composition suivante :

- matière active à tester.......... 400 mg,
- Tween 80........................ 50 mg,
- eau distillée contenant 1 pour 1.000 en poids de Scurol 0..........q.s.p. 40 ml,

en broyant la matière active dans un broyeur de Potter puis en la mettant en suspension dans l'eau contenant le Tween 80 et le Scurol 0.

Le Tween 80 est un agent mouillant constitué par du monolaurate de sorbitan polyoxyéthyléné.

BAD ORIGINAL

Le Scurol O est un agent tensioactif constitué d'alkylphénols polyoxyéthylés, principalement d'octylphénol polyoxyéthylé par 10 moles d'oxyde d'éthylène.

La suspension aqueuse décrite plus haut est ensuite, si nécessaire, diluée par de l'eau distillée contenant 1 pour 1.000 de Scurol O, de façon à obtenir la concentration voulue.

On utilise des pots de diamètre 6,5 cm et de hauteur 8 cm contenant pour moitié de la terre argilolimoneuse et pour moitié du sable de rivière.

Dans chaque pot, on sème 30 graines de l'une des espèces végétales ci-après :

| | symboles |
|---|---|
| Blé (Triticum sativum), variété Caton... | TRIT |
| Vulpin (Avena fatua)..................... | AVEN |
| Lentille (Lens culinaris), variété Large blonde................................. | LENS |
| Radis (Raphanus sativus), variété Rond rose à bout blanc...................... | RAPH |
| Betterave potagère (Beta vulgaris), variété Cerès............................ | BETA |
| Laitue (Lactuca sativa), variété gotte jaune d'or............................. | LAIT |
| Sarrasin (Polygonum fagopyrum).......... | FAG |
| Amarante (Amarantus caudatus)........... | AMA |

Le traitement de prélevée est effectué en pulvérisant sur la surface ensemencée la suspension aqueuse de la matière active, à la concentration voulue, chaque pot recevant environ 0,3 ml de la suspension aqueuse.

Après traitement, on laisse sécher la surface ensemencée puis on recouvre les graines d'une couche de terre de 2 mm environ d'épaisseur.

BAD ORIGINAL

Deux fois par jour, on procède à un arrosage des pots par aspersion et on les maintient en serre à une température de 22 à 24°C, sous une humidité relative de 70 à 80 %, sous un éclairage artificiel apportant environ 5.000lux au niveau des plantes, pendant 16 heures consécutives par 24 heures.

Vingt et un jour après le traitement, on note le nombre et la hauteur des plantes vivantes dans les pots traités par la matière active testée et également le nombre et la hauteur des mêmes plantes dans les pots témoins qui ont été traités selon les mêmes conditions, sans matière active.

On détermine ainsi :

- le pourcentage de réduction en nombre N %, calculé comme suit :

$$N\ \% = \frac{\text{nombre de plantes vivantes dans les pots traités}}{\text{nombre de plantes vivantes dans les pots témoins}} \times 100$$

- le pourcentage de réduction en hauteur H %, calculé comme suit :

$$H\ \% = \frac{\text{hauteur moyenne des plantes vivantes dans les pots traités}}{\text{hauteur moyenne des plantes vivantes dans les pots témoins}} \times 100$$

A partir de N % et H %, on calcule le POURCENTAGE PAR RAPPORT AU TEMOIN, égal à :

$$\frac{N\ \% \times H\ \%}{100}$$

Les valeurs ainsi obtenues sont consignées dans le tableau (I) situé à la fin de la description. Dans ce tableau, une valeur égale à 0 signifie une activité herbicide complète et une valeur égale à 100 signifie l'absence totale d'activité herbicide.

BAD ORIGINAL

Exemple II - Activité herbicide en serre, en post-levée des espèces végétales

On opère selon la méthode décrite dans l'exemple I avec toutefois les différences suivantes :

- après avoir semé les graines, on les recouvre d'une couche de terre de 5 mm d'épaisseur environ, on place les pots dans la serre et on laisse germer les graines de façon à obtenir des plantules au stade de développement voulu,

- le traitement au moyen des composés selon l'invention est effectué lorsque les plantes en sont au stade suivant :

. stade 2 à 3 feuilles pour blé, vulpin, folle-avoine, laitue et amarante,

. stade 3 feuilles vraies pour lentilles,

. stade 2 feuilles cotylédonaires bien développées pour sarrasin, betterave et radis ;

- selon ce traitement de post-levée (ou post-émergence selon une dénomination équivalente), la solution ou suspension contenant la matière active est pulvérisée sur la plante et on laisse ensuite sécher les plantes.

Comme dans le cas de l'exemple précédent, les résultats sont observés vingt et un jours après le traitement et on détermine ainsi le pourcentage par rapport au témoin selon la méthode décrite dans l'exemple précédent.

Les valeurs ainsi déterminées sont consignées dans le tableau II figurant à la fin de la description.

En opérant exactement comme indiqué précédemment, c'est-à-dire en serre, en post-levée des espèces végétales, on a évalué l'activité herbicide du composé n° 1A en mélange avec le composé n° 1B sur un nombre plus important de cultures et d'adventices, et à des doses plus faibles.

Les valeurs ainsi déterminées sont consignées dans le tableau II bis figurant à la fin de la description.

BAD ORIGINAL

Dans ces essais de post-levée, on a observé que le traitement au moyen des composés selon l'invention entraînait un blocage très rapide de la croissance des plantes sensibles. Du fait de ce blocage de la croissance, certaines de ces plantes se sont desséchées rapidement après l'application du produit et étaient mortes au moment du relevé (trois semaines après application du produit). D'autres plantes, dont la croissance a été complètement bloquée par le produit n'étaient pas détruites au moment du relevé mais sont mortes deux à trois semaines après le relevé. Dans les tableaux II et II bis, ces plantes ont généralement été notées de 10 à 20 % puisque la destruction n'était pas complète au moment du relevé, mais on peut considérer que cette destruction serait devenue complète au bout d'un délai supplémentaire de deux à trois semaines.

Exemple III - Activité herbicide et sélectivité vis-à-vis des cultures en serre, en prélevée des espèces végétales

On prépare une poudre mouillable ayant la composition pondérale ci-après :

- composé n° 2A...................... 50 %
- alkylnaphtalène-sulfonate de sodium (Galoryl HT 701)................... 5 %
- acides sulfoniques condensés sous forme de sels de sodium (Galoryl QT 201).. 3 %
- silice antimottante................. 8,8 %
- kaolinite.......................... 33,2 %

et une suspension aqueuse ayant la composition ci-après :

- mélange (55/45) des composés n° 1A et 1B.. 3,6 g
- atlox 4855 (agent émulsionnant à base d'alkylarylsulfonate de calcium et d'un ester d'acide gras polyéthoxylé........... 4 ml
- eau distillée.........q.s.p............... 1 litre

Cette poudre mouillable et cette suspension sont ensuite diluées par de l'eau de façon à obtenir des bouillies ayant la concentration désirée.

BAD ORIGINAL

Dans cet essai, les plantes sont cultivées dans des bacs carrés de 28,5 cm de côtés et 8 cm de profondeur, contenant pour moitié de la terre limoneuse/argileuse et pour moitié du sable de rivière.

Dans chaque bac, on sème en rangées parallèles plusieurs espèces végétales à raison d'une espèce par rangée et on recouvre les graines d'une épaisseur de 4 mm environ de terre.

Le traitement est effectué au moyen d'une rampe de pulvérisation animée d'un mouvement de va-et-vient de façon à pulvériser la bouillie préparée précédemment, à la concentration voulue, ce traitement correspondant à un volume de 500 l/ha de bouillie.

Les bacs sont conservés en serre (t : 24 à 26°C - humidité relative 70 à 80 %), sous un éclairage artificiel apportant 5.000 lux au niveau de la plante, pendant 16 heures consécutives sur 24 heures, avec arrosage quotidien par aspersion.

21 jours après le traitement, on évalue le nombre et la hauteur des plantes vivantes dans chaque bac traité au moyen des composés selon l'invention et on compare aux résultats obtenus dans le cas de bacs contenant les mêmes plantes, mais traités par de l'eau distillée ne contenant pas de matière active.

A partir de ces résultats, on détermine les pourcentages par rapport aux témoins selon la méthode décrite dans l'exemple I. Ces résultats sont consignés dans le tableau (III) figurant à la fin de la description.

Cet exemple n° III illustre tout particulièrement les propriétés herbicides vis-à-vis des mauvaises herbes et la sélectivité vis-à-vis des cultures des composés n° 1A et 1B, testés sous la forme d'un mélange (55/45) de ces deux composés. On observera que ce mélange présente, pour des

BAD ORIGINAL

doses allant de 0,25 à 2 kg/ha une excellente sélectivité vis-à-vis du maïs, du riz et du tournesol, tout en présentant pour ces mêmes doses une bonne activité herbicide vis-à-vis de la plupart des mauvaises herbes traitées.

Exemple IV : Activité herbicide et sélectivité, en serre, en prélevée des espèces végétales

Cet essai est effectué en opérant selon la méthode décrite dans l'exemple précédent, en cultivant les plantes dans des bacs contenant pour moitié de la terre riche en matières organiques (3,7 %) et pour moitié du sable de rivière.

L'essai a été effectué sur les plantes ci-après :

Cultures :

Maïs (Zea maïs)

Tournesol (Helianthus annuus)

Adventices (mauvaises herbes) :

- Vulpin (Alopecurus myosuroïdes)
- Panisse (Echinochloa crus galli)
- Chenopode (Chenopodium album)
- Herbe dure (Sida spinosa)
- Setaire (Setaria viridis)

Les résultats obtenus sont consignés dans le tableau IV figurant à la fin de la description.

D'après ces résultats, on observera que les composés n° 1 A, 8 A, 18 A et 22 A présentent, à des doses allant de 0,250 kg/ha à 1 kg/ha une excellente activité herbicide sur la plupart des mauvaises herbes prises en considération, tout en étant parfaitement toléré par les cultures de Maïs et Tournesol.

Compte-tenu de ces essais, il a été observé que parmi les composés appartenant à la sous-famille préférée mentionnée plus haut, les composés pour lesquels :

- n est égal à 1 ou à 2,
- n est égal à zéro,
- $R_1$ représente un atome de fluor, chlore ou brome ou un radical méthyle, méthoxyle ou méthylène-dioxyle présentaient souvent de remarquables propriétés herbicides sensiblement supérieures à celles des autres membres de la famille.

Les formes A de ces composés, éventuellement en mélange avec les formes B correspondantes sont donc tout particulièrement préférées, tels que, par exemple, les composés n° 1 A, 5 A, 6 A, 8 A, 12 A, 14 A +14 B, 18 A, 22 A, 23 A + 23 B, 27 A et 28 A.

Pour leur emploi dans la pratique, les composés selon l'invention sont en général utilisés sous la forme de compositions également comprises dans le cadre de la présente invention.

Ces compositions comprennent habituellement, en plus de la matière active (composé de formule I), un ou plusieurs supports (ou diluants), solides ou liquides, acceptables en agriculture et un ou plusieurs agents tensio-actifs également acceptables en agriculture. Si nécessaire, elles peuvent contenir de plus divers additifs également acceptables en agriculture.

Habituellement, ces compositions comprennent environ de 0,05 % à 99 % en poids de matière active, d'environ 0 % à 20 % en poids de un ou plusieurs agents tensio-actifs et d'environ 1 % à 99,99 % en poids de un ou plusieurs supports (ou diluants) liquides ou solides.

Comme supports solides, on peut utiliser des silices naturelles ou synthétiques telles que par exemple la terre de diatomée ; des argiles et des silicates naturels ou synthétiques tels que par exemple le talc, l'attapulgite, la vermiculite, les kaolinites, la montmorillonite, les silicates de calcium et d'aluminium ; les carbonates de calcium ; des résines naturelles ou synthétiques telles que le chlorure de polyvinyle .... etc.

BAD ORIGINAL

Comme supports liquides, on peut utiliser l'eau ; les alcools tels que, par exemple l'isopropanol et les glycols ; des cétones telles que, par exemple l'acétone, la méthyl-éthyl-cétone, la méthyl-isobutyl-cétone, la cyclohexanone ... ; des éthers ; des hydrocarbures aromatiques ou araliphatiques tels que le benzène, le toluène, les xylènes, les fractions de pétrole par exemple le kérosène, des hydrocarbures chlorés tels que le tétrachlorure de carbone, le perchloréthylène, etc..

Lorsque le support est de l'eau ou un solvant organique usuel, les compositions selon l'invention comprennent avantageusement en plus de la matière active un agent tensioactif ou une autre matière active à propriétés herbicides.

L'agent tensioactif peut être un agent émulsionnant, dispersant, défloculant ou mouillant, de type ionique ou de type non ionique.

Comme agents tensio-actifs, on peut utiliser avantageusement des polycondensats d'oxyde d'éthylène avec des acides gras ou avec des alcools gras ou avec des phénols substitués (notamment avec des alkyl-phénols ou avec des aryl-phénols) ou avec des amides gras, ou avec des amines grasses. On peut également utiliser avantageusement des esters d'acides gras et du sorbitan, des dérivés du saccharose, des sels de métaux alcalins ou alcalino terreux d'acides lignosulfoniques, d'acides alkylarylsulfoniques ; des sels d'esters d'acides sulfosucciniques ; des esters phosphoriques d'alcools ou phénols polyoxyéthylés ; des alkylbétaïnes ou sulfobétaïnes, etc...

L'ouvrage "Mc Cutcheon's Detergents and Emulsifiers 1975 Annual" MC Publ. Corp. Ridgewood, New Jersey USA décrit de nombreux agents tensio-actifs et donne des recommandations pour leur utilisation.

Le brevet des Etats-Unis d'Amérique n° 3 713 804 décrit de nombreux agents tensio-actifs anioniques ou cationiques ou non ioniques utilisables en agriculture. L'agent

BAD ORIGINAL

tensio-actif à utiliser dans les compositions selon l'invention peut avantageusement être choisi par l'homme de métier parmi les composés ou familles de composés décrits dans ces deux documents.

En plus de la matière active, du support et de l'agent tensioactif, les compositions selon l'invention peuvent contenir d'autres additifs tels que des agents dispersants non tensioactifs, des peptisants, colloïdes protecteurs, des épaississants, des adhésifs augmentant la résistance à la pluie, des stabilisants, des agents conservateurs, des inhibiteurs de corrosion, des colorants, des séquestrants, des anti-mousses, anti-mottants, anti-gels etc...

Les compositions selon l'invention peuvent être sous formes assez diverses, solides ou liquides.

Comme formes de compositions solides, on peut citer les poudres pour poudrage (à teneur en matière active pouvant aller jusqu'à 100 %) et les granulés et microgranulés, notamment ceux obtenus par extrusion, par compactage, par imprégnation d'un support granulé, par granulation à partir d'une poudre (la teneur en matière active dans ces granulés étant entre 1 et 80 % pour ces derniers cas). Ces formes solides sont généralement utilisées à sec, sans dilution ultérieure.

Comme formes de compositions liquides, ou destinées à constituer des compositions liquides lors de l'application, on peut citer les poudres mouillables (ou poudres à pulvériser), les solutions ou concentrés émulsionnables, les suspensions concentrées (ou flowables), les solutions, en particulier les concentrés solubles dans l'eau, les émulsions et dispersions.

Les poudres pour poudrage contiennent habituellement de 0,1 % à 5 % en poids de matière active, un support inerte qui peut être un support d'imprégnation tel qu'un silice et, lorsque nécessaire, de 0 à 10 % en poids de un ou plusieurs stabilisants et/ou d'autres additifs tels que des agents de pénétration, des adhésifs, des agents anti-mottants ou des colorants. Elles sont habituellement préparées par

BAD ORIGINAL

prémélange de la matière active avec le support inerte et le mélange est ensuite broyé dans un broyeur.

Les granulés et micro-granulés, généralement à faible titre de matière active, peuvent être pré-fabriqués puis imprégnés ou fabriqués dans la masse par exemple par atomisation ou par extrusion. Dans ce dernier cas, ils nécessitent l'adjonction de liants et de produits tensio-actifs pour assurer leur délitage rapide au sol. Ils contiennent, en général, de 0,5 à 10 % en poids de matière active, de 0 à 10 % en poids d'additifs comme des stabilisants, des agents de modification à libération lente, des liants et des solvants.

Les poudres mouillables (ou poudre à pulvériser) sont habituellement préparées de manière qu'elles contiennent 20 à 95 % de matière active, et elles contiennent habituellement, en plus du support solide, de 0 à 5 % d'un agent mouillant, de 3 à 10 % d'un agent dispersant, et, quand c'est nécessaire, de 0 à 10 % d'un ou plusieurs stabilisants et/ou autres additifs, comme des agents de pénétration, des adhésifs, ou des agents antimottants, colorants, etc...

A titre d'exemple, voici une composition d'une poudre mouillable à 50 % :

- matière active.................... 50 %
- lignosulfonate de sodium.......... 5 %
- dibutylnaphtalène sulfonate de sodium 1 %
- argile (kaolin).................. 44 %

Un autre exemple de poudre mouillable à la composition suivante :

- matière active.................... 50 %
- lignosulfonate de sodium et/ou de calcium (défloculant)............. 5 %
- isopropylnaphtalène sulfonate (agent mouillant anionique)......... 1 %
- silice antimottante............... 5 %
- kaolin (charge)................... 39 %

BAD ORIGINAL

Pour obtenir ces poudres à pulvériser ou poudres mouillables, on mélange intimement la matière active dans des mélangeurs appropriés avec les substances additionnelles et on broie avec des moulins ou autres broyeurs appropriés. On obtient ainsi des poudres à pulvériser dont la mouillabilité et la mise en suspension sont avantageuses ; on peut les mettre en suspension avec de l'eau à toute concentration désirée et cette suspension est utilisable très avantageusement en particulier pour l'application sur les feuilles des végétaux .

Les solutions ou concentrés émulsionnables contiennent la matière active dissoute dans un solvant (généralement un hydrocarbure aromatique) et en plus, quand c'est nécessaire, un solvant auxiliaire ou cosolvant pouvant être une cétone, un ester, un éther-oxyde, etc...

En général, ils contiennent de 5 à 60 % en poids/volume de matières actives, de 2 à 20 % en poids/volume d'agent émulsifiant et peuvent être préparés, par exemple, par dissolution de matière active, dans le solvant ou dans le mélange de solvants et émulsifiants, dans une cuve équipée d'une bonne agitation et d'une circulation de fluide pour le chauffage ou le refroidissement.

A partir de ces solutions ou concentrés émulsionnables, on peut obtenir par dilution par de l'eau des émulsions de toute concentration désirée qui conviennent particulièrement pour la mise en oeuvre des composés selon l'invention.

Les suspensions concentrées (ou "flowables") sont constituées par une suspension de fines particules solides de la matière active dans l'eau ou une huile non solvante, préparées de manière à obtenir un produit fluide stable, ne se déposant pas. Elles contiennent habituellement de 10 à 75% en poids de matière active, de 0,5 à 15 % d'agents tensioactifs, de 0,1 à 10 % d'agents thixotropes, de 0 à 10 % d'additifs appropriés comme des anti-mousses, des inhibiteurs de corrosion, des stabilisants, des agents de pénétration et des adhésifs et, comme support, de l'eau ou un liquide organique

BAD ORIGINAL

dans lequel la matière active est peu ou pas soluble : certaines matières solides organiques ou des sels minéraux peuvent être dissous dans le support pour aider à empêcher la sédimentation ou comme antigels pour l'eau.

Ces suspensions concentrées sont préparées en broyant très finement la matière active, puis en dispersant la matière active dans le support liquide contenant les ingrédients auxiliaires, puis la dispersion obtenue est broyée dans un broyeur à billes. A partir de suspensions concentrées, on peut obtenir par dilution par de l'eau des suspensions de toute concentration désirée.

Ces suspensions ou émulsions aqueuses de la matière active décrites précédemment, par exemple les compositions obtenues en diluant par de l'eau une poudre mouillable, ou un concentré émulsionnable, ou une suspension concentrée, sont comprises dans le cadre général des compositions selon l'invention.

Les émulsions peuvent être du type eau-dans-l'huile ou huile-dans-l'eau et elles peuvent avoir une consistance épaisse comme celle d'une "mayonnaise".

La présente invention concerne enfin un procédé de désherbage sélectif de cultures, notamment de cultures de maïs et tournesol. Ce procédé est caractérisé en ce qu'il consiste à appliquer sur le sol, en prélevée desdites cultures une quantité efficace vis-à-vis des mauvaises herbes, mais non phytotoxique vis-à-vis des cultures d'un composé selon la formule (I). La quantité de matière active à utiliser peut varier selon le composé utilisé, le type de cultures, les conditions climatiques, la nature des mauvaises herbes à détruire. Dans le cas où on utilise comme matière active les composés préférés mentionnés plus haut, des doses allant de 0,250 kg/ha à 4 kg/ha donnent habituellement de bons résultats.

BAD ORIGINAL

TABLEAU (I)

Activité herbicide en serre en prélevée

Pourcentage par rapport aux témoins

100 : identique au témoin - Aucune activité herbicide
0 : activité herbicide totale

PLANTES

| Composés n° | Dose kg/ha | TRIT | ALOP | AVEN | LENS | RAPH | BETA | LACT | FAG | AMA |
|---|---|---|---|---|---|---|---|---|---|---|
| 1A+1B (55/45) | 6 | 0 | 0 | 7 | 0 | 0 | 0 | 5 | 11 | 0 |
| 1A+1B (55/45) | 2 | 0 | 0 | 8 | 0 | 0 | 0 | 10 | 4 | 0 |
| 2A | 3 | 0 | 21 | 86 | 0 | 0 | 0 | 100 | 100 | 100 |
| 2B | 3 | 76 | 37 | 100 | 6 | 75 | 6 | 100 | 100 | 100 |
| 3A | 3 | 8 | 100 | 100 | 0 | 5 | 0 | 100 | 4 | 6 |
| 3B | 10 | 62 | 38 | 88 | 0 | 70 | 9 | 100 | 100 | 100 |
| 4A | 10 | 63 | 69 | 86 | 80 | 100 | 100 | 100 | 100 | 100 |
| 5A+5B (80/20) | 3 | 0 | 0 | 5 | 0 | 0 | 0 | 19 | 50 | 0 |
| 5A+5B (80/20) | 1 | 12 | 0 | 70 | 0 | 15 | 0 | 100 | 100 | 8 |
| 6A | 3 | 0 | 0 | 12 | 3 | 1 | 8 | 45 | 14 | 0 |
| 6B | 10 | 100 | 27 | 100 | 0 | 100 | 7 | 100 | 100 | 100 |
| | 3 | 100 | 100 | 100 | 20 | 100 | 100 | 100 | 100 | 100 |
| 7A | 3 | 100 | 28 | 100 | 100 | 100 | 50 | 44 | 100 | 100 |
| 8A | 3 | 0 | 0 | 0 | 0 | 0 | 0 | 0 | 10 | 0 |
| 8B | 3 | 21 | 5 | 80 | 0 | 20 | 0 | 59 | 91 | 0 |
| 9A | 3 | 70 | 59 | 100 | 100 | 100 | 86 | 100 | 100 | 100 |
| 10B | 3 | 64 | 100 | 100 | 75 | 100 | 55 | 100 | 100 | 12 |

TABLEAU (I) - (Suite)

Activité herbicide en serre en prélevée

Pourcentage par rapport aux témoins

100 : identique au témoin - Aucune activité herbicide
0 : activité herbicide totale

PLANTES

| Composés n° | Dose kg/ha | TRIT | ALOP | AVEN | LENS | RAPH | BETA | LACT | FAG | AMA |
|---|---|---|---|---|---|---|---|---|---|---|
| 11A | 10 | 71 | 100 | 100 | 0 | 66 | 0 | 100 | 100 | 100 |
| 11B | 10 | 89 | 18 | 100 | 0 | 100 | 10 | 15 | 100 | 100 |
| 12A | 10 | 1 | 1 | 26 | 0 | 0 | 0 | 71 | 100 | 21 |
| 12A | 1 | 19 | 18 | 76 | 0 | 65 | 8 | 100 | 100 | 17 |
| 13A | 3 | 4 | 3 | 34 | 0 | 13 | 0 | 50 | 80 | — |
| 14A + 14B | 3 | 0 | 0 | 1 | 0 | 0 | 0 | 0 | 43 | 0 |
| | 1 | 0 | 3 | 18 | 0 | 0 | 0 | 3 | 85 | 0 |
| 15A + 15B | 10 | 0 | 2 | 29 | 0 | 0 | 0 | 35 | 71 | 0 |
| 16A | 3 | 100 | 36 | 100 | 100 | 100 | 100 | 100 | 100 | 100 |
| 17A | 10 | 11 | 27 | 100 | 0 | 30 | 0 | 100 | 100 | 0 |
| 18A | 3 | 0 | 1 | 4 | 0 | 1 | 0 | 72 | 85 | — |
| 19A | 10 | 75 | 33 | 91 | 3 | 95 | 3 | 100 | 100 | 67 |
| 20B | 10 | 100 | 50 | 100 | 60 | 100 | 80 | 100 | 100 | 100 |
| 21B | 10 | 8 | 32 | 100 | 0 | 66 | 19 | 100 | 100 | 100 |
| 22A | 3 | 0 | 0 | 15 | 0 | 0 | 0 | 3 | 9 | 0 |
| 23A + 23B | 3 | 0 | 0 | 0 | 0 | 0 | 0 | 0 | 6 | 0 |

TABLEAU (I) - (Suite)

Activité herbicide en serre en prélevée

Pourcentage par rapport aux témoins

100 : identique au témoin - Aucune activité herbicide
0 : activité herbicide totale

PLANTES

| Composés n° | Dose kg/ha | TRIT | ALOP | AVEN | LENS | RAPH | BETA | LACT | FAG | AMA |
|---|---|---|---|---|---|---|---|---|---|---|
| 24A + 24B | 10 | 3 | 13 | 100 | 0 | 32 | 0 | 100 | 100 | 0 |
| 25B | 1 | 43 | 82 | 45 | 100 | 100 | 100 | 100 | 100 | 0 |
| 26A | 1 | 10 | 40 | 80 | 0 | 0 | 0 | 100 | 100 | 100 |
| 26B | 3 | 74 | 48 | 100 | 0 | 61 | 43 | 100 | 83 | 100 |
| 27A | 1 | 0 | 3 | 22 | 0 | 38 | 2 | 34 | 32 | 0 |
| 27B | 3 | 78 | 0 | 46 | 8 | 100 | 10 | 92 | 100 | 0 |
| 28A | 3 | 0 | 0 | 2 | 0 | 5 | 0 | 10 | 41 | 0 |
| 28B | 10 | 38 | — | 19 | 0 | 100 | 6 | 67 | 100 | 0 |
| 29A | 10 | 0 | 0 | 2 | 0 | 0 | 0 | 19 | 7 | 0 |
| 29B | 10 | 30 | — | 76 | 0 | 100 | 8 | 100 | 100 | 100 |

TABLEAU II

Activité herbicide en serre en postlevée

Pourcentage par rapport aux témoins

100 % : identique au témoin - aucune activité herbicide
0 % : activité herbicide totale.

| Composé n° | Dose kg/ha | TRIT | ALOP | AVEN | LENS | RAPH | BETA | LACT | FAG | AMA |
|---|---|---|---|---|---|---|---|---|---|---|
| | | PLANTES | | | | | | | | |
| 1A+1B (55/45) | 6 | 35 | 0 | 100 | 2 | 40 | 0 | 80 | 100 | 25 |
| 1A+1B (55/45) | 2 | 50 | 10 | 100 | 35 | 60 | 0 | 100 | 100 | 25 |
| 3B | 10 | 100 | 100 | 100 | 30 | 100 | 4 | 75 | 100 | 100 |
| 3B | 3 | 100 | 100 | 100 | 90 | 100 | 16 | 75 | 100 | 100 |
| 5A+5B (80/20) | 3 | 60 | 9 | 100 | 28 | 45 | 0 | 45 | 16 | 13 |
| 5A+5B (80/20) | 1 | 68 | 48 | 100 | 82 | 80 | 50 | 60 | 47 | 50 |
| 6B | 10 | 100 | 100 | 100 | 37 | 100 | 14 | 100 | 100 | 100 |
| | 3 | 100 | 100 | 100 | 75 | 100 | 11 | 100 | 100 | 100 |
| 8A | 3 | 27 | 0 | 100 | 23 | 5 | 0 | 13 | 19 | 80 |
| 8B | 3 | 100 | 100 | 91 | 87 | 100 | 13 | 100 | 61 | 100 |

TABLEAU (II) bis

Activité herbicide, en serre postlevée

Pourcentages par rapport aux témoins

100 : identique au témoin - aucune activité herbicide
0 : activité herbicide totale

Composé testé : mélange 1A + 1B (55/45)

| Doses kg/ha | 1 | 0,5 |
|---|---|---|
| Plantes : | | |
| Betterave (Beta vulgaris) | 0 | 0 |
| Blé (Triticum sativum) | 70 | 100 |
| Orge (Hordeum sativum) | 80 | 100 |
| Maïs (Zea maïs) | 100 | 100 |
| Tournesol (Helianthus annus) | 100 | 100 |
| Laitue (Lactuca sativa) | 100 | 100 |
| Sarrasin (Polygonum fagopyrum) | 100 | 100 |
| Vulpin (Alopecurus myosuroïdes) | 70 | 80 |
| Folle avoine (Avena fatua) | 60 | 100 |
| Panisse (Echinochloa crus-galli) | 60 | 100 |
| Setaire (Setaria germanica) | 30 | 100 |
| Amarante (Amarantus caudatus) | 30 | 100 |
| Ipomée (Ipomea purpurea) | 20 | 50 |
| Bleuet (Centaura cyanus) | 20 | 50 |
| Chrysanthème (Chrysanthemum annuum | 10 | 30 |
| Morelle noire (Solanum nigrum) | 60 | 60 |
| Coquelicot (Papaver rhoeas) | 10 | 20 |

TABLEAU (III)

Activité herbicide en serre en prélevée

Pourcentages par rapport aux témoins

| PLANTES | Composé n° | | | | |
|---|---|---|---|---|---|
| | 1A + 1B (55/45) | | | 2A | |
| Dose kg/ha | 0,25 | 1 | 2 | 0,5 | 1 |
| Maïs (Zea maïs) | 100 | 100 | 100 | 93 | 63 |
| Riz (Orizae sativa) | 100 | 100 | 100 | 78 | 78 |
| Tournesol (Helianthus annuus) | 100 | 100 | 100 | 94 | 70 |
| Laitue (Lactuca sativa) | 100 | 88 | 59 | | |
| Coton (Gossypium; sp) | 100 | 100 | 67 | 100 | 100 |
| Soja (Glycina hispida) | 100 | 15 | 8 | 100 | 41 |
| Haricot (Phaseolus vulgaris) | 100 | 100 | 67 | | |
| Lin (Linium) | | | | 100 | 100 |
| Folle-avoine (Avena fatua) | 100 | 55 | 23 | 100 | 85 |
| Vulpin (Alopecurus myosuroïdes) | 73 | 7 | 0 | | |
| Panisse (Echinochloa crus galli) | 100 | 29 | 0 | | |
| Digitaire (Digitaria sanguinalis) | 12 | 0 | 0 | | |
| Stellaire (Stellarid media) | 0 | 0 | 0 | 7 | 0 |
| Chenopode (Chenopodium album) | 60 | 0 | 0 | 80 | 19 |
| Capselle bourse à pasteur (Capsella bursa-pastoris) | 60 | 0 | 0 | 16 | 0 |
| Herbe dure (Sida spinosa) | 59 | 2 | 0 | 3 | 0 |
| Plantain lanceole (Plantago lanceolata) | 0 | 0 | 0 | 4 | 0 |

TABLEAU (IV)

Activité herbicide en serre, en prélevée

Pourcentages par rapport au témoins

| | Composé n° | | | | | | | |
|---|---|---|---|---|---|---|---|---|
| | 1 A | | 8 A | | 18 A | | 22 A | |
| Dose kg/ha | 0,25 | 1 | 0,25 | 1 | 0,25 | 1 | 0,5 | 1 |
| Culture | | | | | | | | |
| Zea maïs | 100 | 100 | 100 | 100 | 100 | 100 | 100 | 100 |
| Helianthus annuus | 100 | 100 | 100 | 100 | 100 | 100 | 100 | 100 |
| Adventices | | | | | | | | |
| Alopecurus myosuroïdes | 53 | 0 | 10 | 9 | 73 | 8 | 18 | 0 |
| Echinochloa cus galli | 56 | 0 | 15 | 4 | 67 | 2 | 26 | 0 |
| Stellaria media | 0 | 0 | 0 | 0 | 5 | 0 | 7 | 0 |
| Chenopodium album | 30 | 0 | 0 | 0 | 13 | 0 | 0 | 0 |
| Sida spinosa | 8 | 0 | 0 | 0 | 100 | 100 | 70 | 0 |
| Setaria viridis | 62 | 2 | 0 | 0 | 100 | 25 | 40 | 1 |
| Viola cornuta | 50 | 0 | 0 | 0 | 25 | 21 | 10. | 0 |
| Phalaris paradoxa | 25 | 3 | 3 | 0 | 15 | 7 | 28 | 0 |

TABLEAU (V)

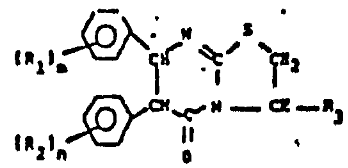

| No. composé | 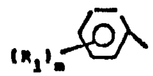 | 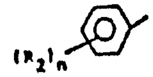 | $R_3$ | Forme | Point de fusion (°C) |
|---|---|---|---|---|---|
| 6 A | 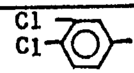 | 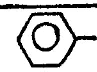 | H | A | 126-130 |
| 9 A | 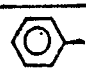 | 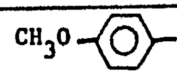 | H | A | 109 |
| 11 A<br>11 B | 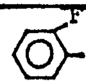 | 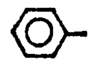 | H | A<br>B | 209<br>172 |
| 12 A | 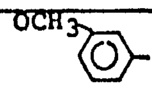 | 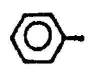 | H | A | 116 |
| 13 A | 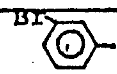 | 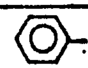 | H | A | 120-122 |
| 14 A + 14 B | 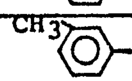 | 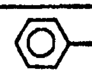 | H | A + B<br>(90/10) | 132 |
| 15 A + 15 B | 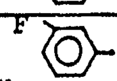 | 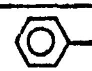 | H | A + B<br>(80/20) | 133 |
| 16 A | 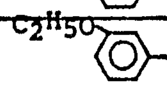 | 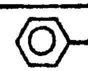 | H | A | 151 |
| 17 A | 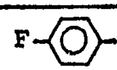 | 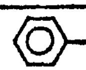 | H | B | 168 |
| 18 A | 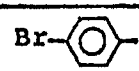 | 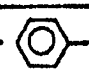 | H | A | 159 |
| 19 A | 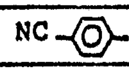 | 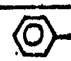 | H | A | 193 |

TABLEAU (V) - (Suite)

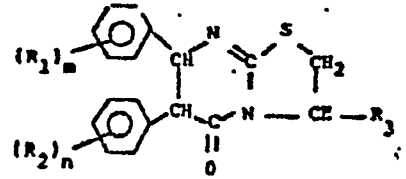

| No. composé | 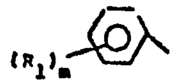 | 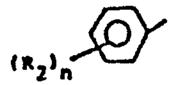 | $R_3$ | Forme | Point de fusion (°C) |
|---|---|---|---|---|---|
| 20 B | 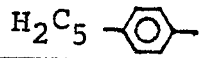 | 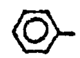 | H | B | 167 |
| 21 B | 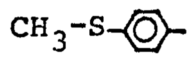 | 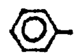 | H | B | 147 |
| 22 A | 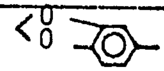 | 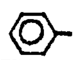 | H | A | 177 |
| 23 A + 23 B | 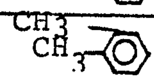 | 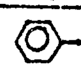 | H | A + B (95/5) | 132 |
| 24 A + 24 B | 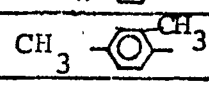 | 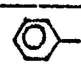 | H | A + B (85/15) | 173 |
| 25 B | 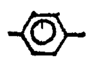 | 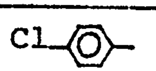 | H | B | 172 |
| 26 A<br>26 B | 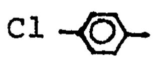 | 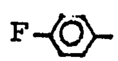 | H | A<br>B | 194<br>146 |
| 27 A<br>27 B | 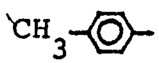 | 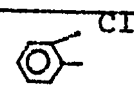 | H | A<br>B | 136<br>181 |
| 28 A<br>28 B | 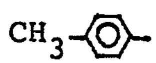 | 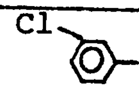 | H | A<br>B | 121<br>187 |
| 29 A<br>29 B | 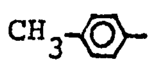 | 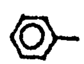 | $-CH_3$ | A<br>B | 113<br>196 |

REVENDICATIONS

1) Dérivé de la tétrahydro-2,3,6,7 5H-thiazolo [3,2-a] pyrimidine caractérisé en ce qu'il répond à la formule générale (I) :

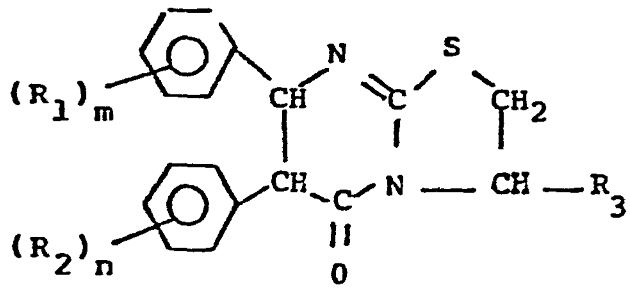

(I)

dans laquelle :

$R_1$ représente un atome d'halogène, un radical alkyle comportant au plus 6 atomes de carbone, éventuellement mono ou polyhalogéné ; alkoxyle comportant au plus 6 atomes de carbone ; alkylthio comportant au plus 6 atomes de carbone ; nitro ; cyano ; amino ; alkylamino comportant au plus 6 atomes de carbone, dialkylamino dans lequel chacune des parties alkyle, identiques ou différentes, comporte de 1 à 4 atomes de carbone ; acétamido ; R-CO dans lequel R représente un radical alkyle comportant au plus six atomes de carbone, éventuellement mono ou polyhalogéné, ou deux substitutants $R_1$ forment ensemble un radical divalent alkylènedioxyle comportant au plus 6 atomes de carbone ;

m est égal à 0, 1, 2, 3, 4 ou 5 étant entendu que les substituants $R_1$ peuvent être identiques ou différents,

$R_2$ représente un atome d'halogène, un radical alkyle comportant au plus 6 atomes de carbone éventuellement mono ou polyhalogéné ou un radical alkoxyle comportant au plus 6 atomes de carbone, nitro ou cyano,

n est égal à 0, 1, 2 ou 3 étant entendu que les substituants peuvent être soit identiques soit différents,

BAD ORIGINAL

$R_3$ représente l'atome d'hydrogène ou un radical alkyle comportant au plus six atomes de carbone, et formes stéréoisomères de ce dérivé ainsi que mélanges de ces formes.

2) Composé selon la revendication 1 caractérisé en ce qu'il répond à la formule (I) dans laquelle $R_1$ représente un atome d'halogène ou un radical alkyle comportant de 1 à 3 atomes de carbone, alkoxyle comportant de 1 à 3 atomes de carbone, ou un radical alkylènedioxyle comportant de 1 à 3 atomes de carbone, m est égal à 0, 1 ou 2, $R_2$ représente un radical alkyle comportant de 1 à 3 atomes de carbone, n est égal à 0 ou à 1 et $R_3$ représente l'atome d'hydrogène, ou le radical méthyle.

3) Procédé de préparation d'un composé selon la revendication 1, caractérisé en ce que l'on fait réagir le dérivé d'acide, de formule (II) :

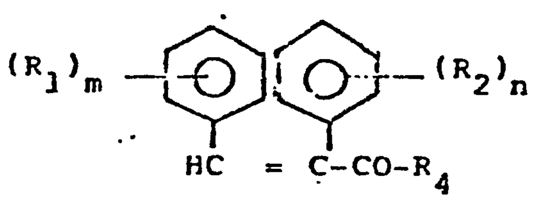

(II)

dans laquelle les différents symboles ont la même signification que dans la revendication 1 et $R_4$ représente un atome de chlore ou un radical alkoxyle inférieur, sur l'imino-2 thiazolidine de formule (III) :

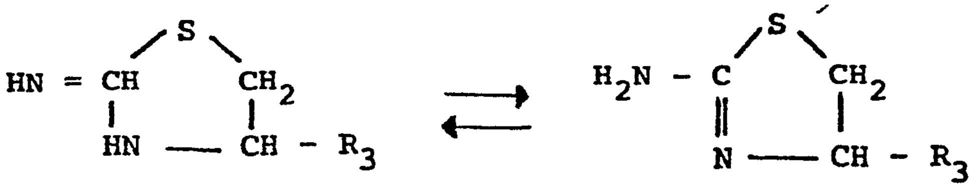

(III) (IV)

BAD ORIGINAL

en équilibre avec sa forme amino-2 thiazoline-2, de formule (IV) et en ce que le produit formé est séparé du mélange réactionnel.

4) Procédé selon la revendication 3, caractérisé en ce que l'on fait réagir le chlorure d'acide répondant à la formule (II) dans laquelle $R_4$ représente l'atome de chlore, sur l'imino-2 thiazolidine de formule (III), en équilibre avec sa forme amino-2 thiazoline-2 (IV) à une température comprise entre moins 40°C et plus 160°C, en présence d'un accepteur d'acide, en milieu solvant inerte de préférence anhydre.

5) Procédé de préparation de la forme A du composé selon la revendication 1, à partir de la forme B de ce composé, caractérisé en ce que l'on traite ladite forme B par une base, minérale ou organique, en milieu solvant organique, à une température comprise entre plus 15°C et plus 80°C.

6) Procédé selon la revendication 5, caractérisé en ce que la base est un alcoolate de métal alcalin et le solvant est un alcanol ou un éther.

7) Procédé selon la revendication 5, caractérisé en ce que la base est la soude ou la potasse et le solvant un alcanol.

8) Composition herbicide caractérisée en ce qu'elle comprend comme matière active un composé selon la revendication 1.

9) Composition selon la revendication 8, caractérisée en ce que, en plus de la matière active, elle comprend un ou plusieurs supports solides ou liquides acceptables en agriculture et un ou plusieurs agents tensio-actifs acceptables en agriculture.

BAD ORIGINAL

10) Procédé de désherbage sélectif de cultures de maïs, riz, tournesol ou coton caractérisé en ce que l'on applique sur le sol, en prélevée de ces cultures une quantité efficace mais non phytotoxique à l'égard de la culture, d'un composé selon la revendication 1.

BAD ORIGINAL

REVENDICATIONS POUR L'AUTRICHE

1) Composition herbicide, caractérisée en ce qu'elle comprend comme matière active un dérivé de la tétrahydro-2,3,6,7 5H-thiazolo [3,2-a] pyrimidine répondant à la formule générale (I)

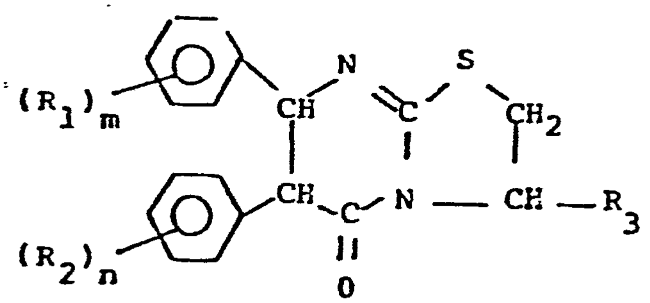

(I)

dans laquelle :

$R_1$ représente un atome d'halogène, un radical alkyle comportant au plus 6 atomes de carbone, éventuellement mono ou polyhalogéné ; alkoxyle comportant au plus 6 atomes de carbone ; alkylthio comportant au plus 6 atomes de carbone ; nitro ; cyano ; amino ; alkylamino comportant au plus 6 atomes de carbone, dialkylamino dans lequel chacune des parties alkyle, identiques ou différentes, comporte de 1 à 4 atomes de carbone ; acétamido ; R-CO dans lequel R représente un radical alkyle comportant au plus six atomes de carbone, éventuellement mono ou polyhalogéné, ou deux substitutants $R_1$ forment ensemble un radical divalent alkylènedioxyle comportant au plus 6 atomes de carbone ;

m est égal à 0, 1, 2, 3, 4 ou 5 étant entendu que les substituants $R_1$ peuvent être identiques ou différents,

$R_2$ représente un atome d'halogène, un radical alkyle comportant au plus 6 atomes de carbone éventuellement mono ou polyhalogéné ou un radical alkoxyle comportant au plus 6 atomes de carbone, nitro ou cyano,

n est égal à 0, 1, 2 ou 3 étant entendu que les substituants peuvent être soit identiques soit différents,

BAD ORIGINAL

$R_3$ représente l'atome d'hydrogène ou un radical alkyle comportant au plus six atomes de carbone, ou une forme stéréoisomère de ce dérivé seule ou en mélange avec une autre forme stéréoisomère de ce dérivé.

2) Composition selon la revendication 1, caractérisée en ce qu'elle contient comme matière active un composé répondant à la formule (I) dans laquelle $R_1$ représente un atome d'halogène ou un radical alkyle comportant de 1 à 3 atomes de carbone, alkoxyle comportant de 1 à 3 atomes de carbone, ou un radical alkylènedioxyle comportant de 1 à 3 atomes de carbone, m est égal à 0, 1 ou 2, $R_2$ représente un radical alkyle comportant de 1 à 3 atomes de carbone, n est égal à 0 ou à 1 et $R_3$ représente l'atome d'hydrogène, ou le radical méthyle.

3) Composition selon l'une des revendications 1 et 2, caractérisée en ce que, en plus de la matière active, elle comprend un ou plusieurs supports solides ou liquides acceptables en agriculture et un ou plusieurs agents tensio-actifs acceptables en agriculture.

4) Procédé de préparation d'un composé selon la formule générale (I)

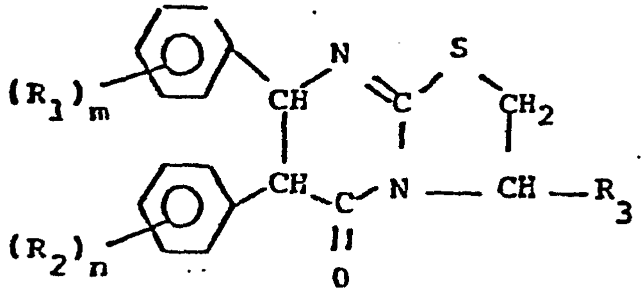

(I)

dans laquelle $R_1$, $R_2$, m, n et $R_3$ ont la même signification que dans la revendication 1, caractérisé en ce que l'on fait réagir le dérivé d'acide, de formule (II) :

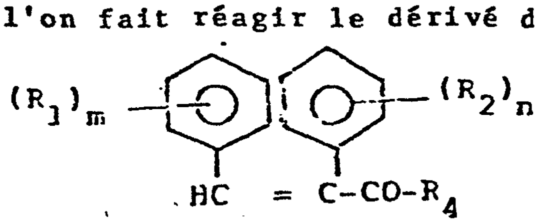

(II)

BAD ORIGINAL

dans laquelle les différents symboles ont la même signification que précédemment et $R_4$ représente un atome de chlore ou un radical alkoxyle inférieur, sur l'imino-2 thiazolidine de formule (III) :

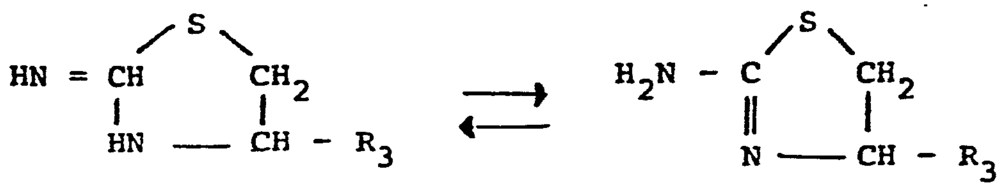

(III) (IV)

en équilibre avec sa forme amino-2 thiazoline-2, de formule (IV) et en ce que le produit formé est séparé du mélange réactionnel.

5) Procédé selon la revendication 4, caractérisé en ce que l'on fait réagir le chlorure d'acide répondant à la formule (II) dans laquelle $R_4$ représente l'atome de chlore, sur l'imino-2 thiazolidine de formule (III), en équilibre avec sa forme amino-2 thiazoline-2 (IV) à une température comprise entre moins 40°C et plus 160°C, en présence d'un accepteur d'acide, en milieu solvant inerte de préférence anhydre.

6) Procédé de préparation de la forme A du composé selon la formule (I) décrite dans la revendication 4, à partir de la forme B de ce composé, caractérisé en ce que l'on traite ladite forme B par une base minérale ou organique, en milieu solvant organique, à une température comprise entre plus 15°C et plus 80°C.

7) Procédé selon la revendication 6, caractérisé en ce que la base est un alcoolate de métal alcalin et le solvant est un alcanol ou un éther.

8) Procédé selon la revendication 7, caractérisé en ce que la base est la soude ou la potasse et le solvant un alcanol.

9) Procédé de désherbage sélectif de cultures de maïs, riz, tournesol ou coton, caractérisé en ce que l'on applique sur le sol, en prélevée de ces cultures une quantité efficace mais non phytotoxique à l'égard de la culture, d'une composition selon la revendication 1.

0102908

Office européen des brevets

# RAPPORT DE RECHERCHE EUROPEENNE

Numéro de la demande

EP 83 42 0112

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| D,A | EP-A-0 045 251 (RHONE-POULENC)<br>* Revendication 1 *<br>----- | 1 | C 07 D 513/04<br>A 01 N 43/90 //<br>(C 07 D 513/04<br>C 07 D 277/00<br>C 07 D 239/00 ) |
| | | | **DOMAINES TECHNIQUES RECHERCHES (Int. Cl. ³)** |
| | | | C 07 D 513/00<br>A 01 N 43/00 |

Le présent rapport de recherche a été établi pour toutes les revendications

| Lieu de la recherche | Date d'achèvement de la recherche | Examinateur |
|---|---|---|
| LA HAYE | 27-10-1983 | ALFARO I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant